Europäisches Patentamt

European Patent Office

Office européen des brevets

(19)

(11) Numéro de publication: **0 208 637**
**A1**

## DEMANDE DE BREVET EUROPEEN

(21) Numéro de dépôt: 86430021.5

(22) Date de dépôt: 18.06.86

(51) Int. Cl.⁴: **C12N 15/00**

(30) Priorité: 19.06.85 FR 8509429

(43) Date de publication de la demande:
14.01.87 Bulletin 87/03

(84) Etats contractants désignés:
AT BE CH DE FR GB IT LI LU NL SE

(71) Demandeur: **CENTRE NATIONAL DE LA RECHERCHE SCIENTIFIQUE (CNRS)**
**15, Quai Anatole France**
**F-75007 Paris(FR)**

(72) Inventeur: **Teissie, Justin**
**1 bis rue du Salas**
**F-31520 Ramonville Sainte Agne(FR)**

(74) Mandataire: **Rinuy, Santarelli**
**14, avenue de la Grande Armée**
**F-75017 Paris(FR)**

(54) Procédé perfectionné de fusion cellulaire.

(57) L'invention concerne un procédé perfectionné de fusion cellulaire essentiellement caractérisé par le fait qu'il consiste à créer un tapis cellulaire continu par filtration d'un milieu de culture cellulaire au travers d'un filtre et à soumettre ce tapis cellulaire humide à l'action des impulsions d'un champ électrique.

## Procédé perfectionné de fusion cellulaire

La présente invention concerne un procédé perfectionné pour obtenir une fusion cellulaire.

On sait que la fusion cellulaire permet l'obtention d'espèces hybrides somatiques à valeur économique accrue. Une telle fusion constitue donc un outil de choix pour l'industrie bio-technologique.

Les différentes méthodes connues d'hybridations reposent essentiellement sur l'usage d'agents chimiques (du type Polyéthylèneglycol = PEG) ou viraux (origine : Virus de Sendai).

Depuis quelques années, on a préconisé de tirer profit de la perforation des membranes cellulaires induite par des champs électriques intenses et de brève durée.

Il est de toute façon, quelle que soit la technique utilisée, indispensable qu'un contact intime soit créé entre les cellules. Dans le cas de cellules en suspension, les méthodes proposées font appel à des additifs chimiques du type rappelé ci-dessus (PEG) qui ont très souvent une action létale, ou bien à des traitements au moyen d'enzymes protéolytiques (comme la pronase) qui sont susceptibles de pénétrer dans le cytoplasme lors de la pulsion électrique.

Or, la présente invention se propose de fournir un procédé faisant également appel à des impulsions électriques mais ne faisant, en aucun cas, intervenir d'agents chimiques.

Le procédé selon la présente invention est essentiellement caractérisé par le fait qu'il consiste à créer un tapis cellulaire continu par filtration d'un milieu de culture cellulaire au travers d'un filtre et à soumettre ce tapis cellulaire humide à l'action des impulsions d'un champ électrique .

Suivant d'autres caractéristiques :

-le filtre utilisé pour la mise en oeuvre du procédé ci-dessus est en une matière non cyto-toxique pour la souche cellulaire en cause ;

-la taille des pores du filtre utilisé est compatible avec la dimension et la rhéologie des cellules à retenir;

-le champ électrique appliqué pour créer les impulsions voulues est réalisé de façon connue en soi, la création, l'entretien et la durée des impulsions étant à la portée de l'homme de l'art, en fonction du type de cellules auquel ce champ est appliqué ;

-les impulsions du champ électrique sont appliquées parallèlement au plan longitudinal du tapis de cellules retenues sur le filtre.

Le demandeur a constaté qu'en procédant comme indiqué ci-dessus, on obtenait, avec un bon rendement, une fusion cellulaire sans faire appel à des agents chimiques et en utilisant des moyens connus pour engendrer simplement un champ électrique.

Les seules précautions à prendre pour la mise en oeuvre de ce procédé sont celles relatives au choix de la matière constitutive du filtre au travers duquel est filtrée la culture cellulaire en cause.

Bien entendu, ce choix sera fait de manière qu'aucune action létale ne soit constatée sur les cellules.

Il va de soi que la taille des pores du filtre choisi sera fonction de la taille et de la rhéologie des cellules à retenir.

On veillera également à conserver sur le filtre supportant les cellules séparées de leur milieu de culture un taux d'humidité suffisant pour "maintenir en vie" ces cellules en vue de leur fusion.

La description qui va suivre ainsi que l'illustration par des exemples du procédé de l'invention feront mieux apprécier l'intérêt et la portée de celle-ci.

Comme exposé précédemment, l'invention fait appel à un certain volume d'une suspension cellulaire que l'on fait écouler librement à travers un filtre pour retenir les cellules. On comprend que de cette façon on peut réaliser un tapis cellulaire continu sur le filtre par le choix judicieux de la taille de ce filtre, du volume et de la densité volumique de la culture cellulaire. En effet, du fait de la réduction des dimensions du volume offert aux cellules au cours de cette opération de filtration, leur densité se trouve considérablement accrue, si bien que le volume libre offert à chaque cellule chute également considérablement si bien que de nombreux contacts se créent entre les cellules, contacts éminemment recherchés pour leur fusion. De plus, on notera que les cellules séparées de leur milieu peuvent être rincées avec tout milieu approprié. Il est, en effet, connu dans le cas des cellules poussant sur un support, que le rendement de la fusion induite par un champ électrique dépend éminemment de la nature des ions présents dans le milieu. On notera également que la durée de l'écoulement peut être contrôlée de manière à conserver une fine pellicule de liquide imbibant le tapis cellulaire sur le filtre. Il est, en effet, essentiel, comme le comprendra l'homme de l'art, de ne pas amener à sec les cellules.

Conformément à l'invention, le filtre portant son tapis cellulaire, humide, sera alors soumis à des impulsions d'un champ électrique réalisé parallèlement au plan horizontal du tapis cellulaire, champ dont les caractéristiques seront choisies comme cela est bien connu dans la technique, pour favoriser le rendement de fusion optimum. Les caractéristiques des impulsions du champ sont, comme on le sait, fonction du type cellulaire.

Après incubation dans le milieu de culture, on constate que les cellules sont fusionnées et viables.

L'intérêt du procédé selon l'invention réside, comme déjà signalé, dans l'absence de tout additif chimique susceptible d'intéragir avec les cellules et de modifier, de manière irréversible, leur comportement.

Il est ainsi possible de tirer profit, dans le cas de cellules en suspension, de tous les avantages bien connus des cellules adhérant à des supports solides (rendement élevé, obtention d'hybrides somatiques, facilité de manipulation).

L'exemple suivant est donné à titre illustratif et nullement limitatif de mise en oeuvre du procédé sur des cellules d'ovaires d'hamsters chinois.

Le matériel de départ est une culture de cellules d'ovaires d'hamsters chinois en suspension - (culture sous agitation). La densité cellulaire est de $10^6$ cellules par ml.

Le filtre est en polycarbonate (matériau non cytotoxique) avec des trous calibrés (diamètre 1 ou 2μm) ; son diamètre est de 25 mm. Il peut être du type vendu par la Société NUCLEPORE. Le filtre est installé dans un support de filtration du type vendu, par exemple, par la Société GELMAN. Cet ensemble peut éventuellement être stérilisé.

Le filtre est prélavé avec 2 ml de milieu de culture (MEM 0111 complété en sérum, antibiotiques et glutamine). L'écoulement est libre par simple gravitation. 1 ml de la culture cellulaire est alors filtré par écoulement libre (l'écoulement a lieu jusqu'à ce que le délai entre chaque goutte soit supérieur à 10 secondes). 2 ml de milieu dit de pulsation sont alors filtrés (le milieu de pulsation est un tampon phosphate 10mM pH 7,2, 1mM Mg + +, 250mM saccharose). Les conditions d'écoulement sont les mêmes que les précédentes.

Aux termes des manipulations décrites ci-dessus, on a formé un tapis cellulaire sur le filtre, le liquide interstitiel étant le milieu de pulsation.

Le filtre est alors déposé sur une boîte de culture (Marque NUNC, diamètre 37 mm). Deux électrodes parallèles distantes de 18 mm sont amenées en contact avec le filtre de telle façon qu'une surface de 324 mm2 (soit 18x18mm) soit disposée entre les électrodes. Les cellules placées sur cette surface seront traitées lors de l'application d'une tension de 2 kV (soit un champ de 1,1kV/cm) pendant une durée de 100 μs entre les électrodes décrites ci-dessus. Ces décharges électriques sont répétées 5 fois de suite (avec des délais d'environ 1 seconde). Les électrodes sont alors retirées et un volume de 2ml de milieu de culture est déposé dans la boîte. La boîte est ensuite placée dans un incubateur pendant une heure à la température du 37°C. Les cellules sont alors décollées du fil tre par agitation de celui-ci après l'avoir retourné. Les cellules sont enfin mises en suspension dans le milieu de culture présent dans la boîte.

Leur observation en microscopie en contraste de phase révèle la présence de cellules multinucléées illustrant la fusion cellulaire car une telle multinucléation n'est pas présente chez les cellules qui n'ont subi aucun traitement (culture de départ) ou qui ont subi tout le traitement décrit ci-dessus main où aucune décharge électrique n'a été appliquée.

L'ensemble de toutes ces opérations est réalisé de façon stérile sous une hotte à flux laminaire.

Il va du reste de soi que la présente invention n'a été décrite qu'à titre purement explicatif et nullement limitatif et que toute modification utile au niveau des équivalences pourra y être apportée sans sortir de son cadre.

## Revendications

1. Procédé perfectionné de fusion cellulaire essentiellement caractérisé par le fait qu'il consiste à créer un tapis cellulaire continu par filtration d'un milieu de culture cellulaire au travers d'un filtre et à soumettre ce tapis cellulaire humide à l'action des impulsions d'un champ électrique.

2. Procédé selon la revendication 1, caractérisé en ce que le filtre utilisé pour sa mise en oeuvre est en une matière non cyto-toxique pour la souche cellulaire en cause.

3. Procédé selon la revendication 1 ou 2, caractérisé en ce que la taille des pores du filtre utilisé est compatible avec la dimension et la rhéologie des cellules à retenir.

4. Procédé selon l'une quelconque des revendications 1 à 3, caractérisé en ce que le champ électrique appliqué pour créer les impulsions voulues est réalisé de façon connue en soi.

5. Procédé selon la revendication 4, caractérisé en ce que les impulsions du champ électrique sont appliquées parallèlement au plan longitudinal du tapis de cellules retenues sur le filtre.

## DOCUMENTS CONSIDERES COMME PERTINENTS

| Catégorie | Citation du document avec indication. en cas de besoin. des parties pertinentes | Revendication concernee | CLASSEMENT DE LA DEMANDE (Int Cl 4) |
|---|---|---|---|
| A | NATURWISSENSCHAFTEN, vol. 67, 1980, pages 414,415, Springer Verlag, DE; E. NEUMANN et al.: "Cell fusion induced by high electric impulses applied to Dictyostelium" * En entier * | 1-5 | C 12 N  15/00 |
| A | SCIENCE, vol. 216, no. 4545, 30 avril 1982, pages 537,538, AAAS; J. TEISSIE et al.: "Electric pulse-induced fusion of 3T3 cells in monolayer culture" | | |
| A | EXP. CELL. RES., vol. 150, 1984, pages 477-482, Academic Press Inc.; C. FINAZ et al.: "A new, highly efficient technique for generating somatic cell hybrids" | | |
| P,X | DE-U-8 504 192   (GCA CORP.) * En entier * | 1-4 | |
| P,A | EP-A-0 159 954   (CNRS) * En entier * | 1-5 | |

DOMAINES TECHNIQUES
RECHERCHES (Int. Cl.4)

C 12 N

Le présent rapport de recherche a été établi pour toutes les revendications

| Lieu de la recherche | Date d'achèvement de la recherche | Examinateur |
|---|---|---|
| LA HAYE | 29-09-1986 | DESCAMPS J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur. mais publié à la date de dépôt ou après cette date
D : cité dans la demande
L : cité pour d'autres raisons

& : membre de la même famille. document correspondant

OEB Form 1503 03 82